# EUROPÄISCHE PATENTANMELDUNG

(11) **EP 2 439 670 A2**
(43) Veröffentlichungstag der Anmeldung: **11.04.2012**
(21) Anmeldenummer: 11184717.4
(22) Anmeldetag: 11.10.2011
(51) Int. Cl.: G06F 19/00

(54) **Schreibbrett, Verfahren zur Überprüfung und/oder Beurteilung eines durch vorgegebene Kriterien definierten Sollzustands, Computerprogrammprodukt zur Überprüfung und/oder Beurteilung eines durch vorgegebene Kriterien definierten Sollzustands sowie Verwendung eines Schreibbretts**

(30) Priorität: 11.10.2010 DE 102010038100
(71) Anmelder: Riesinger, Michael, 48153 Münster (DE)
(72) Erfinder: Riesinger, Michael, 48153 Münster (DE); Jansing, Frank, 48301 Nottuln (DE)
(74) Vertreter: Michalski Hüttermann & Partner

(57) **Zusammenfassung**

Ein Schreibbrett (10), insbesondere Klemmbrett, zur manuellen Informationsspeicherung weist eine Schreibplatte (12) zur Bereitstellung einer Schreibfläche und mindestens eine mit der Schreibplatte (12) verbundene Messeinrichtung (14, 22, 24) zur Ermittlung von Pflegedaten und/oder Versorgungsdaten für die Pflege- und/oder Versorgungssituation eines Menschen, insbesondere Menschen in geriatrischer und/oder palliativmedizinischer Behandlung, auf. Die Überprüfung der Pflege- und/oder Versorgungssituation kann dadurch schneller und sicherer erfolgen.

## Beschreibung

Die Erfindung betrifft ein Schreibbrett, Verfahren zur Überprüfung und/oder Beurteilung eines durch vorgegebene Kriterien definierten Sollzustands, ein Computerprogrammprodukt zur Überprüfung und/oder Beurteilung eines durch vorgegebene Kriterien definierten Sollzustands sowie eine Verwendung eines Schreibbretts, mit deren Hilfe bei der Überprüfung eines Sollzustands verschiedene Kriterien abgeprüft und verarbeitet werden können.

Insbesondere bei Pflegeinstitutionen zur Pflege von Menschen, die sich in geriatrischer und/oder palliativmedizinischer Behandlung befinden, besteht ein Bedürfnis, die Qualität der Pflege- und/oder Versorgungssituation für die Menschen zu überprüfen. Hierzu ist es bekannt, mit dem Leitungspersonal der Pflegeinstitution und/oder mit entsprechenden Pflegekräften in einem Gespräch einen Fragenkatalog durchzugehen und zu überprüfen, ob die gestellten Fragen zur Pflege- und/oder Versorgungssituation der Menschen befriedigend beantwortet werden können. Durch die Fragen des Fragenkatalogs werden bestimmte Kriterien abgeprüft, die einen wünschenswerten Sollzustand definieren. Auf Grundlage der Anzahl befriedigender Antworten wird das Ausmaß des Erreichens des Sollzustands bewertet. Nachteilig bei einem derartigen Verfahren ist, dass die Beurteilungsgrundlage für die Bewertung auf nicht ausreichend fundierten oder nicht vollständig zutreffenden Angaben beruht, so dass die Beurteilung unpräzise ist. Gleichzeitig ist es erforderlich, die Befragung während des normalen Betriebs der Pflegeinstitution durchzuführen, so dass der Zeitaufwand für die beteiligten Personen zur

Durchführung der Befragung möglichst gering gehalten werden muss, um überhaupt eine Befragung durchführen zu können. Der damit verbundene Zeitdruck führt ebenfalls zu Qualitätsdefiziten bei der Überprüfung und/oder Beurteilung der Pflege- und/oder Versorgungssituation der Menschen.

Aus US 2010/0194976 A1 ist ein zu diagnostischen Zwecken verwendetes Schreibbrett bekannt, das einem Menschen mit mehreren Fragen zum körperlichen Wohlbefinden konfrontiert, die von dem Menschen beantwortet werden können. Die Antworten zu diesen Fragen können von dem Schreibbrett aufgenommen und an eine Person weitergeleitet werden, die auf Grundlage der beantworteten Fragen eine ärztliche Diagnose erstellen und medizinische Maßnahmen veranlassen kann.

Insbesondere bei Institutionen, die Pflege und Hilfe für hilfs- und pflegedürftige Personen bereitstellen (Pflegeinstitutionen) besteht ein ständiges Bedürfnis einen durch vorgegebene Kriterien definierten Sollzustand schnell, sicher und nachhaltig überprüfen und/oder beurteilen zu können.

Es ist die Aufgabe der Erfindung Maßnahmen anzugeben, die eine schnelle und sichere Überprüfung und/oder Beurteilung eines durch vorgegebene Kriterien definierten Sollzustands ermöglicht.

Die Lösung der Aufgabe erfolgt erfindungsgemäß durch ein Schreibbrett mit den Merkmalen des Anspruchs 1, durch ein Verfahren zur Überprüfung und/oder Beurteilung eines durch vorgegebene Kriterien definierten Sollzustands mit den Merkmalen des Anspruchs 12, ein Computerprogrammprodukt zur Überprüfung und/oder Beurteilung eines durch vorgegebene Kriterien definierten Sollzustands mit den Merkmalen des Anspruchs 14 sowie eine Verwendung mit den Merkmalen des Anspruchs 15. Bevorzugte Ausgestaltungen der Erfindung sind in den Unteransprüchen angegeben, die jeweils einzeln oder in Kombination einen Aspekt der Erfindung darstellen können.

Das erfindungsgemäße Schreibbrett, insbesondere Klemmbrett, zur manuellen Informationsspeicherung weist eine Schreibplatte zur Bereitstellung einer Schreibfläche auf. Erfindungsgemäß ist mit der Schreibplatte eine Messeinrichtung zur Ermittlung von Pflegedaten und/oder Versorgungsdaten für die Pflege- und/oder Versorgungssituation von Menschen, insbesondere Menschen in geriatrischer und/oder palliativmedizinischer Behandlung, verbunden.

Das Schreibbrett wird insbesondere zu nicht-diagnostischen Zwecken verwendet. Insbesondere werden mit der mindestes einen Messeinrichtung keine medizinischen Werte gemessen, die einen Menschen betreffen. Insbesondere werden mit dem Schreibbrett keine Körperwerte, beispielsweise Blutzuckerwert, Körpertemperatur, Blutdruck, Puls, Blutgruppe etc. gemessen, wobei derartige Körperwerte mit Hilfe des Schreibbretts vorzugsweise auch erst gar nicht messbar sind. Stattdessen wird das Schreibbrett vorzugsweise zur Pflege- und/oder Umgebungsdokumentation in einer Pflegeeinrichtung verwendet, wobei insbesondere das Schreibbrett zu diesem Anwendungszweck speziell hergerichtet ist. Die Messeinrichtung ist insbesondere derart ausgestaltet, dass medizinische Werte auch erst gar nicht erfasst werden können, indem beispielsweise eine als Thermometer ausgestaltete Messeinrichtung derart in die Schreibplatte integriert ist, mit der Schreibplatte verbunden ist und/oder derart dimensioniert ist, dass es eben nicht zum Fibermessen bei einem Menschen verwendet werden kann. Das Schreibbrett ist insbesondere derart ausgestaltet, dass die Ermittlung und/oder Überprüfung von Pflegedaten und/oder Versorgungsdaten auch von nicht ärztlich oder medizinisch ausgebildeten Personen durchgeführt werden kann. Beispielsweise kann ein Krankenpfleger oder ein Gerätetechniker den Zustand und/oder die Einstellungen der für einen Menschen in geriatrischer und/oder palliativmedizinischer Behandlung verwendeten medizinischen Geräte oder die Funktionsweise einer Klimaanlage zur Einstellung eines für einen Menschen in geriatrischer und/oder palliativmedizinischer Behandlung erforderlichen Raumklimas überprüfen.

Mit der Schreibplatte kann beispielsweise eine Klemme zum Festklemmen von Papier verbunden sein, so dass Papierseiten mit mehreren zu stellenden und zu überprüfenden Fragen einfach bereitgestellt werden können. Zusätzlich oder alternativ können ergänzende Anmerkungen, Notizen oder Maßnahmen zur Verbesserung einer Pflege- und/oder Versorgungssituation notiert werden. Diese Informationsspeicherung muss jedoch nicht notwendigerweise konventionell, beispielsweise mit Papier und Bleistift, erfolgen, sondern kann auch elektronisch durchgeführt werden. Hierzu können beispielweise mit Hilfe eines Displays elektronisch gespeicherte Fragen von der Schreibplatte dargestellt werden und über eine geeignete Eingabeeinrichtung die Antworten und/oder ergänzenden Informationen eingegeben werden. Insbesondere weist die Schreibplatte einen Touchscreen auf, mit dessen Hilfe zu beantwortende Fragen dargestellt und die entsprechenden Antworten gespeichert werden können. Besonders bevorzugt werden die Antworten und/oder Messergebnisse nicht lokal im Schreibbrett gespeichert, sondern mit Hilfe eines geeigneten Datenübertragungsmittels, beispielsweise ein drahtlos übertragender Datentransmitter oder Datentransceiver, in einem Server gespeichert, wo die erhaltenen Daten mit älteren gespeicherten Daten verglichen und/oder mit anderen Daten verknüpft werden können. Beispielsweise können in dem Server gemessene Klimadaten innerhalb einer Zimmers mit aktuellen Witterungsdaten verknüpft werden, um einen möglichen Defekt einer Klimaanlage mit einer höheren Genauigkeit identifizieren zu können. Ferner ist es möglich eine zeitliche Entwicklung einer Pflege- und/oder Versorgungssituation eines bestimmten Menschs nachzuhalten, wodurch der Effekt von eingeleiteten Verbesserungsmaßnahmen und/oder eintretende Verschlechterungen frühzeitig und nachhaltig erkannt werden können. Besonders bevorzugt sind in dem Schreibbrett und/oder in dem Server immer wieder benötigte Daten, beispielsweise Bettenanzahl, Anzahl der Einzelzimmer einer geriatrischen Pflegeeinrichtung vorgehalten, so dass diese Daten nicht bei jedem Informationserfassungsprozess erneut erfasst werden müssen. Dies erleichtert und beschleunigt die Erfassung der mit dem Schreibbrett erhältlichen Informationen, wodurch Stress und eine unnötig erhöhte Fehleranfälligkeit bei den Bedienpersonen vermieden wird. Darüber hinaus ist die Wahrscheinlichkeit eines Datenverlusts bei in einem Server gespeicherten Daten reduziert.

Insbesondere im medizinischen Bereich ist es eine anerkannte und übliche Praxis, mit Hilfe eines Klemmbretts bei einer Visite personenbezogene Daten, beispielsweise Patientendaten, bereitzuhalten und ergänzende Informationen zu notieren. Das erfindungsgemäße Schreibbrett kann dadurch entsprechend einfach in die übliche Arbeitsroutine, beispielsweise einer Pflegeinstitution integriert werden, indem aus dem medizinischen Bereich bekannte Arbeitsabläufe in den Pflegebereich, insbesondere in den geriatrischen Bereich, übertragen werden. Hierbei wird die Erkenntnis ausgenutzt, dass in einer geriatrischen Pflegeeinrichtung beispielsweise im Gegensatz zu Hygieneanforderungen in einem Operationssaal eines Krankenhauses zur Überprüfung einer Pflege- und/oder Versorgungssituation fachärztliches Spezialwissen nicht erforderlich ist und die Übertragung von Arbeitsweisen aus dem medizinischen Bereich in den geriatrischen Bereich überraschenderweise kostengünstig und effizient erfolgen kann.

Mit Hilfe der mindestens einen mit der Schreibplatte befestigten Messeinrichtung können bei einer Visite schnell und effizient allgemeine Messdaten, beispielsweise über das Raumklima im Bewohnerzimmer und/oder des Wohnumfelds mit hoher Genauigkeit ermittelt werden. Dadurch ist es nicht erforderlich, durch eine Befragung von Personen die Erfüllung von Kriterien für einen bestimmten Sollzustand, beispielsweise Kriterien über das Raumklima, erfragen zu müssen. Die Abprüfung derartiger Kriterien kann dadurch genauer und sicherer erfolgen. Unnötige Fehler und Ungenauigkeiten können vermieden werden. Ein Zusatzaufwand, während der Befragung mit Hilfe von erst zu beschaffenden Messgeräten Messungen durchzuführen, wird dadurch vermieden. Ferner kann die Befragung von Personen auf wenige Kriterien reduziert werden, wodurch die Überprüfung und Bewertung der Erreichung eines Sollzustands schneller und nachhaltiger erfolgen kann. Eine Störung der Arbeitsroutine durch die Befragung kann dadurch minimiert werden.

Insbesondere ist die mit der Schreibplatte verbundene mindestens eine Messeinrichtung auf den speziellen Verwendungszeck ausgestaltet im geriatrischen Bereich Informationen zu erfassen. Dadurch ist es möglich die Messeinrichtung auf die zur Pflege- und/oder Versorgungssituation von Menschen in geriatrischer und/oder palliativmedizinischer Behandlung relevanten Daten zu beschränken, die sich beispielsweise aus einem vorgegebenen Kriterienkatalog ergeben. Dieser Kriterienkatalog kann beispielsweise durch ein Qualitätsmanagement und/oder gesetzliche Vorgaben vorgegeben werden. Beispielsweise kann der Sollzustand, insbesondere einzuhaltende Mindeststandards, für eine Pflegesituation und/oder eine Umgebungssituation für eine Pflegeeinrichtung durch Kriterien definiert werden, die freiwillig oder gesetzlich vorgegeben sein können, wobei freiwillig vorgegebene Qualitätskriterien insbesondere gesetzlich vorgegebene Kriterien übertreffen, um eine Pflege- und/oder Umgebungssituation nachhaltig zu ermöglichen, welche gesetzlich vorgesehene Mindeststandards übertreffen können. Insbesondere kann die Messeinrichtung dahingehend ausgestaltet sein, dass lediglich die Erfüllung beziehungsweise Nicht-Erfüllung eines bestimmten Kriteriums erfasst wird. Insbesondere wird durch die Messeinrichtung nicht eine zu messende Größe quantitativ ermittelt sondern lediglich qualitativ mit einem Soll-Wert verglichen. Dies vereinfacht den Aufbau der Messeinrichtung und erleichtert die Auswertung der erfassten Informationen, wodurch eine schnelle und sichere Überprüfung und/oder Beurteilung eines durch vorgegebene Kriterien definierten Sollzustands einer Pflege- und/oder Versorgungssituation von Menschen in geriatrischer und/oder palliativmedizinischer Behandlung ermöglicht ist. Die Messeinrichtung ist insbesondere direkt, das heißt unmittelbar ohne weitere zwischengeschaltete Bauteile, mit Hilfe eines Befestigungsmittels mit der Schreibplatte befestigt. Insbesondere ist es mit Hilfe der mit der Schreibplatte verbundenen mindestens einen Messeinrichtung möglich subjektiv zu bewertende weiche Kriterien durch objektiv mit Hilfe der Messeinrichtung überprüfbare harte Kriterien zu ersetzen, wodurch sich eine erhöhte Objektivität und Reliabilität ergibt. Darüber hinaus ist es möglich die Zeit zur Ermittlung der Daten zur Pflege- und/oder Versorgungssituation, insbesondere der Umgebungssituation, schnell und routiniert durchzuführen. Längeres Überlegen, ob ein bestimmtes Kriterium aus subjektiver Sicht erfüllt oder nicht erfüllt erscheint, kann vermieden werden. Dies führt dazu, dass in der Pflegeeinrichtung eingesetzte Personen für die Dokumentation der Pflege- und/oder Versorgungssituation entsprechend weniger Zeit benötigen und für diese Personen mehr bewohnernahe Zeit, beispielsweise für Gespräche und/oder Pflegeleistungen, verbleibt, wodurch das Wohlbefinden der Bewohner der Pflegeeinrichtung gesteigert werden kann.

Insbesondere ist eine Messeinrichtung als Längenmesseinrichtung zur Detektion eines Abstands zwischen einer Oberkante eines Bettgitters eines Bewohnerbetts zu einer Oberseite einer Matratze des Bewohnerbetts ausgestaltet, wobei die Längenmesseinrichtung insbesondere als schwenkbar mit der Schreibplatte verbundene Lehre ausgestaltet ist. Mit Hilfe der Längenmesseinrichtung kann überprüft werden, ob in Bezug zur Matratze des Bewohnerbetts das Bettgitter am seitlichen Rand des Bewohnerbetts eine erforderliche Mindesthöhe ("C-Maß") aufweist, damit der Bewohner des überprüften Bewohnerzimmers nicht versehentlich aus dem Bett fallen kann. Die erforderliche Mindesthöhe kann insbesondere gesetzlich und/oder durch eine Norm vorgegeben sein. Dadurch kann besonders einfach überprüft werden, ob im Falle mehrerer Unterlagen für einen Menschen die Höhe des Bettgitters noch ausreichend gewählt ist oder nachgestellt werden muss. Ein geeigneter Abstand der entgegen der Schwerkraftrichtung weisenden Oberkante des Bettgitters zu der entgegen der Schwerkraftrichtung weisenden Oberseite der Matratze beträgt beispielsweise circa 30 cm. Vorzugsweise ist ein Stab entsprechender Länge oder mit einer Länge, die einem gesetzlich vorgegebenen Mindestmaß für diesen Abstand entspricht, schwenkbar mit der Schreibplatte verbunden, so dass der Stab eine Lehre ausbildet. Die Schreibplatte kann auf der Oberkante des Bettgitters aufgelegt werden, so dass die Einhaltung des entsprechenden Abstands durch das Verschwenken des Stabs überprüft werden kann. Wenn der verschwenkte Stab an der Matratze anschlägt, ist der Abstand nicht ausreichend hoch gewählt und es kann eine Empfehlung abgegeben werden, das Bettgitter höher zu positionieren. Im eingeschwenkten Zustand kann der Stab eine seitliche Kante der Schreibplatte ausbilden und insbesondere oberflächenbündig in den Formverlauf der Schreibplatte integriert sein. Alternativ ist es möglich für die Längenmesseinrichtung ein automatisch aufrollbares Maßband vorzusehen. Ferner ist es möglich, die Länge berührungslos zu messen, beispielsweise mit Hilfe eines Ultraschall- und/oder Infrarot-Sender/Empfängers.

Vorzugsweise ist mit der Schreibplatte ein zwischen einer eingeklappten Ruheposition und einer im Wesentlichen rechtwinklig abstehenden Gebrauchsposition bewegbarer Ansatz verbunden, wobei der Ansatz insbesondere in der Ruheposition reibschlüssig und/oder formschlüssig feststellbar ist. Insbesondere kann der Ansatz in der Ruheposition oberflächenbündig in den Formverlauf der Schreibplatte integriert sein. Durch den herausklappbaren Ansatz kann zwischen der Schreibplatte und dem Ansatz im Wesentlichen ein rechter Winkel ausgebildet werden, der es ermöglicht, die Schreibplatte im Wesentlichen rechtwinklig zu einem Bettgitter eines Bewohnerbetts anzuordnen. Eine Längenmessung eines Abstands zwischen einer Oberkante des Bettgitters eines Bewohnerbetts zu einer Oberseite einer Matratze eines Bewohnerbetts wird dadurch vereinfacht und kann präzisier erfolgen. Zusätzlich oder alternativ kann mit der Schreibplatte eine Wasserwaage verbunden sein, mit deren Hilfe insbesondere eine horizontale und/oder vertikale Ausrichtung der Schreibplatte überprüft werden kann.

Besonders bevorzugt ist eine Messeinrichtung als Thermometer zur Messung der Temperatur eines Bewohnerzimmers und/oder als Feuchtigkeitsmesser zur Luftfeuchtigkeitsmessung des Bewohnerzimmers ausgestaltet. Dadurch können wesentliche Einflussgrößen für das Raumklima des Bewohnerzimmers und/oder des Wohnumfelds, vorzugweise automatisch detektiert werden. Zusätzlich oder alternativ kann der Sauerstoffgehalt des Bewohnerzimmers und/oder des Wohnumfelds gemessen werden. Dadurch ist es beispielsweise möglich, ein ausreichendes Lüften des Bewohnerzimmers feststellen zu können. Ferner ist es möglich, eine zu hoch oder zu niedrig eingestellte Heizleistung einer Heizung feststellen zu können und eine Überprüfung der Einstellungen der Heizkörper und/oder der Funktionsweise der Heizkörper zu empfehlen. Dies ermöglicht es, durch schnelle und einfache Maßnahmen konkrete Maßnahmen anzugeben, um das Wohlbefinden und/oder die Pflege- und/oder Versorgungssituation von Menschen zu verbessern. Insbesondere bei längeren Hitzeperioden im Sommer kann eine zu warme Klimatisierung und eine dadurch mögliche Dehydrierung des Menschen, der das Bewohnerzimmer bewohnt, vermieden werden.

Insbesondere ist eine Messeinrichtung als Fotometer zur Helligkeitsmessung eines Bewohnerzimmers ausgestaltet. Das Fotometer kann beispielsweise ein einfach ausgestaltetes Fotoelement aufweisen, deren Ausgangsstrom gemessen wird. Das Ergebnis der Helligkeitsmessung kann insbesondere automatisch auf einem Display angezeigt werden. Dies ermöglicht es eine zu dunkle oder zu helle Beleuchtung des Bewohnerzimmers und/oder des Wohnumfelds festzustellen und auf ein Ausmaß einzustellen, das zu einem höheren Wohlbefinden des in dem Bewohnerzimmer wohnenden Menschen führt.

Vorzugsweise ist eine Messeinrichtung als Lautstärkemesser zur Lautstärkemessung eines Bewohnerzimmers ausgestaltet. Die Messergebnisse des Lautstärkemessers können insbesondere auf einem Display automatisch angezeigt werden. Dies ermöglicht es, eine mögliche Lärmbelästigung des Bewohners festzustellen und entsprechende Gegenmaßnahmen einzuleiten, um das Wohlbefinden des Bewohners zu verbessern.

In einer bevorzugten Ausführungsform ist mit der Schreibplatte eine Leseeinheit, insbesondere Barcode-Scanner, zur Identifikation von im Bewohnerzimmer vorgesehenen Hilfsmitteln verbunden, wobei die Hilfsmittel jeweils mit einem von der Leseeinheit lesbaren Code versehen sein können. Insbesondere bei Pflegeinstitutionen sind die in einem Bewohnerzimmer und/oder im Wohnumfeld bereitgestellten Hilfsmittel inventarisiert und beispielsweise mit einem Barcode versehen. Entsprechendes gilt für gemietete oder geleaste Hilfsmittel eines Dritten. Mit Hilfe der Leseeinheit kann das jeweilige Hilfsmittel eindeutig identifiziert werden, so dass es beispielsweise möglich ist, für dieses Hilfsmittel hinterlegte Statusinformationen nachzusehen. Dadurch ist es insbesondere möglich festzustellen, ob ein bestimmtes Hilfsmittel gewartet werden muss. Ferner ist es möglich zu überprüfen, ob das jeweilige Hilfsmittel aufgrund des Zustands des Bewohners überhaupt vorgesehen ist und/oder aufgrund der medizinischen Beurteilung einer durchgeführten Visite überhaupt noch benötigt wird. Insbesondere ist es möglich zu überprüfen, ob alle für einen Bewohner vorgesehenen Hilfsmittel in dem Bewohnerzimmer vorhanden sind. Ein fehlendes Hilfsmittel kann dadurch identifiziert werden, so dass die Beschaffung des noch fehlenden Hilfsmittels veranlasst werden kann. Ferner ist es möglich, mit der Leseeinheit einen dem jeweiligen Bewohner und/oder dem jeweiligen Bewohnerzimmer und/oder dem jeweiligen Wohnumfeld zugeordneten Code zu lesen, so dass die Informationen für die identifizierten Hilfsmittel mit Informationen über den Bewohner und/oder das Bewohnerzimmer einfach verknüpft werden können, um beispielsweise eine ausreichende Ausstattung des Bewohnerzimmers und/oder des Wohnumfelds mit Hilfsmitteln schnell und einfach überprüfen zu können.

Insbesondere ist mit der Schreibplatte eine Lampe, vorzugsweise in der Art einer Taschenlampe, verbunden. Durch die Lampe ist es möglich auch das Innere von Geräten zu überprüfen. Ferner ist es möglich eine ausreichende Wundversorgung genau untersuchen zu können. Auch bei unzureichender Beleuchtung, beispielsweise bei einem lichtempfindlichen Bewohner, kann eine sorgfältige Überprüfung der Pflege- und/oder Versorgungssituation durchgeführt werden.

Vorzugsweise weist die Schreibplatte einen Datenaustauschport, beispielsweise einen USB-Anschluss, auf. Dies ermöglicht es beispielsweise mit Hilfe eines über den Datenaustauschport angeschlossenen drahtlosen Datentransceiver, über das Schreibbrett aufgenommene Daten an einen Server zu übertragen und/oder auf einem Server hinterlegte Daten zur verfügung zu stellen.

Besonders bevorzugt weist die Schreibplatte ein Display, insbesondere Touchscreen auf, wobei das Display mit einer Rechnereinheit verbunden ist. Mit Hilfe des Displays können die einzelnen Messergebnisse der Messeinrichtung angezeigt werden. Ferner ist es möglich über das Display einen abzuprüfenden Fragenkatalog abzubilden. Besonders bevorzugt können mit einer entsprechenden Eingabeeinheit, insbesondere über das Display selber, verschiedene Messergebnisse abgefragt werden und/oder verschiedene Fragen durchgeblättert und/oder beantwortet werden. Insbesondere kann ein Großteil einer Oberseite der Schreibplatte als Display ausgestaltet sein.

Besonders bevorzugt ist die Rechnereinheit mit der mindestens einen Messeinrichtung zur Darstellung der Messergebnisse auf dem Display und/oder zur Verarbeitung der Messergebnisse verbunden. Die von der Messeinrichtung gelieferten Messergebnisse werden dadurch nicht nur auf dem Display dargestellt, sondern können zusätzlich von der Rechnereinheit verarbeitet werden. Beispielsweise können abzuprüfende Fragen eines Fragenkatalogs mit Hilfe der gemessenen Messergebnisse automatisch beantwortet werden und/oder eine auf Grundlage der Messergebnisse vorselektierte Antwort markiert werden.

In einer bevorzugten Ausführungsform weist die Rechnereinheit ein Computerprogrammprodukt zur Pflegeüberprüfung und/oder Pflegebeurteilung auf, wobei das Computerprogrammprodukt derart ausgestaltet ist, dass beim Ausführen des Computerprogrammprodukts auf dem Display Fragen zur Pflege- und/oder Versorgungssituation eines Menschen ausgegeben werden, Antworten auf die Fragen detektiert und weiterverarbeitet werden, wobei bei der Weiterverarbeitung der Antworten durch einen Vergleich mit Referenzdaten die Antworten bewertet und nichtbefriedigende Antworten identifiziert werden, und im Falle einer nichtbefriedigenden Antwort mit Hilfe eines gespeicherten Expertensystems Verbesserungsvorschläge unterbereitet werden, wobei die unterbreiteten Verbesserungsvorschläge, insbesondere weitere Fragen aufweisen und auf Grundlage der weiteren Antworten auf die weiteren Fragen mit Hilfe des gespeicherten Expertensystems weitere Verbesserungsvorschläge unterbreitet werden. In den Referenzdaten können die Parameter, deren Vorliegen das Erfüllen eines bestimmten Kriteriums definieren, hinterlegt sein. Hierbei kann es sich auch um Schwellwerte handeln, die zur Erfüllung eines bestimmten Kriteriums überschritten und/oder unterschritten sein müssen. Wenn die Beantwortung einer Frage dazu führt, dass ein bestimmtes Kriterium nicht erfüllt ist, kann die Antwort als nicht befriedigend bewertet werden, sofern die Antworten auf andere Fragen diese Antwort nicht aus anderen Gründen eine Bewertung als befriedigende Antwort rechtfertigen. Die von dem Expertensystem erstellten Vorschläge werden insbesondere nach Beantwortung aller Fragen bereitgestellt, um die Befragung nicht durch umgehend dargestellte Verbesserungsvorschläge zu verlangsamen. Ferner können die Antworten zu anderen Fragen Einfluss auf den zu unterbreitenden Verbesserungsvorschlag haben, so dass es möglich ist sämtliche gesammelten Informationen bei der Erstellung eines Verbesserungsvorschlags zu berücksichtigen. Die Verbesserungsvorschläge können vorzugsweise in einer abschließenden Auswertung zusammengefasst sein und beispielsweise eine ToDo-Liste von durchzuführenden Maßnahmen aufweisen.

Mit Hilfe des Computerprogrammprodukts und insbesondere einer auf einem geeigneten Datenträger gespeicherten Software, welche die Rechnereinheit in die Lage versetzt, derartige Tätigkeiten durchzuführen, können beispielsweise in einem mehrstufigen und/oder iterativen Verfahren verschiedene Probleme identifiziert werden und Lösungsvorschläge zur Lösung der identifizierten Probleme unterbreitet werden. In dem Fall, dass ein bestimmtes Kriterium des definierten Sollzustands nicht ausreichend erfüllt ist, wird der Nutzer nicht mit dieser Feststellung alleingelassen, sondern es werden mit Hilfe des Expertensystems Hinweise und Verbesserungsvorschläge unterbreitet. Dies erhöht die Akzeptanz einer durchgeführten Überprüfung, da dadurch nicht die Beurteilung der beteiligten Personen, sondern eine Verbesserung der aktuellen Situation zur Erreichung eines wünschenswerten Sollzustands im Vordergrund steht. Es ist dadurch möglich, nicht nur eine Qualitätsmessung, sondern auch eine Qualitätsverbesserung einfach und schnell herbeizuführen. Das Expertensystem weist insbesondere einen gespeicherten Maßnahmenkatalog auf, der auf Grundlage der Erfahrungen von Experten berücksichtigt, welche Gründe für die Nicht-Erfüllung eines bestimmten Kriteriums erfahrungsgemäß vorliegen. Insbesondere wenn die gesammelten Informationen ein nicht ganz stimmiges Gesamtbild ergeben, kann auch die Hinzuziehung eines bestimmten Experten vorgeschlagen werden, so dass auch unübliche und ungewöhnliche Situationen berücksichtigt werden können.

Insbesondere ist die Rechnereinheit mit einem Transmitter zur drahtlosen Kommunikation erhaltener Daten mit einer Servereinheit verbunden, wobei insbesondere die Rechnereinheit zur permanentspeicherlosen Übertragung der erhaltenen Daten mit dem Transmitter verbunden ist. Die mit Hilfe der Messeinrichtung erhaltenen Messdaten können dadurch auf einem Server gespeichert werden, so dass bei einer Wiederholung der Überprüfung Veränderungen zu früheren Messungen identifiziert werden können. Ferner ist es möglich, in Reaktion auf den abgearbeiteten Fragenkatalog Maßnahmen zur Verbesserung des Istzustands zur Erreichung des Sollzustands zu veranlassen und hierbei auf eine aktualisierte und gesicherte Datenbasis zurückzugreifen. Besonders bevorzugt werden die erhaltenen Daten nicht permanent gespeichert, sondern lediglich in einem flüchtigen Speicher zwischengespeichert. Dadurch ist es möglich, den Datenschutz zu verbessern und insbesondere personenbezogene Daten oder Daten, die einer bestimmten Person zugeordnet werden können, vor einem unberechtigten Zugriff besser zu schützen. Besonders bevorzugt können die erhaltenen Daten nach einer definierten Zeitspanne automatisch gelöscht werden, so dass es nicht möglich ist, die entsprechenden Daten im Falle eines versehentlich liegen gelassenen Schreibbretts durch Unbefugte auslesen zu lassen.

Besonders bevorzugt weist das Schreibbrett eine Kamera, insbesondere Digitalkamera, zur Aufnahme von Bildern und/oder Filmen auf. Die Kamera kann vorzugsweise ein Objektiv aufweisen, dessen Außenseite seitlich zur Schreibplatte ausgerichtet ist. Die Außenseite des Objektivs kann insbesondere formschlüssig in den Formverlauf einer die Schreibplatte begrenzenden Seitenfläche integriert sein. Die Kamera kann insbesondere mit der Rechnereinheit und/oder einem permanenten und/oder flüchtigen Speicher verbunden sein. Mit der Kamera können beispielsweise zu Dokumentationszwecken Fotographien aufgenommen werden. Diese Fotographien können als Bilder in eine Auswertung und/oder ein Gutachten eingebettet werden, um durch entsprechendes Bildmaterial und/oder Filmmaterial beachtenswerte Aspekte zur Pflege- und/oder Versorgungssituation eines Menschen in einer Pflegeeinrichtung verdeutlichen zu können. Eine spätere Auswertung kann dadurch auf genaueren und verlässlicheren Daten erfolgen. Insbesondere kann bei einer mehrmaligen Überprüfung der Pflege- und/oder Versorgungssituation des selben Menschen eine Verbesserung und/oder Verschlechterung leichter festgestellt werden. Dies erleichtert es Veränderungen einer Pflege- und/oder Versorgungssituation eines Menschen nachhalten und dokumentieren zu können.

Die Erfindung betrifft ferner ein Verfahren zur Überprüfung und/oder Beurteilung eines durch vorgegebene Kriterien definierten Sollzustands, insbesondere einer geriatrischen und/oder palliativmedizinischen Pflege- und/oder Versorgungssituation eines Menschen, bei dem auf einem Display auf Basis der vorgegebenen Kriterien Fragen zum Istzustand ausgegeben werden, Antworten auf die Fragen detektiert und weiterverarbeitet werden, wobei bei der Weiterverarbeitung der Antworten durch einen Vergleich mit Referenzdaten die Antworten bewertet und nichtbefriedigende Antworten identifiziert werden, und im Falle einer nichtbefriedigenden Antwort mit Hilfe eines gespeicherten Expertensystems Verbesserungsvorschläge unterbreitet werden, wobei die unterbreiteten Verbesserungsvorschläge insbesondere weitere Fragen aufweisen und auf Grundlage der weiteren Antworten auf die weiteren Fragen mit Hilfe des gespeicherten Expertensystems weitere Verbesserungsvorschläge unterbreitet werden. Dies ermöglicht eine schnelle, sichere und nachhaltige Überprüfung der Erreichung eines Sollzustands, wobei insbesondere bei nichtausreichend erfüllten Kriterien mit Hilfe des Expertensystems geeignete Verbesserungsvorschläge unterbreitet werden können. Das Verfahren kann insbesondere wie vorstehend anhand des Schreibbretts erläutert aus- und weitergebildet sein.

Das Verfahren wird insbesondere zu nicht-diagnostischen Zwecken angewendet. Insbesondere werden durch die vorgegeben Kriterien keine medizinischen Werte vorgegeben, die einen Menschen betreffen. Beispielsweise kann ein Krankenpfleger oder ein Gerätetechniker anhand der vorgegeben Kriterien den Zustand und/oder die Einstellungen der für einen Menschen in geriatrischer und/oder palliativmedizinischer Behandlung verwendeten medizinischen Geräte oder die Funktionsweise einer Klimaanlage zur Einstellung eines für einen Menschen in geriatrischer und/oder palliativmedizinischer Behandlung erforderlichen Raumklimas überprüfen. Insbesondere werden zur Überprüfung und/oder Beurteilung des Sollzustands relevante Messdaten mit Hilfe eines Schreibbretts ermittelt, das wie vorstehend beschrieben aus- und weitergebildet sein kann. Zusätzlich oder alternativ werden die Ausgabe der Fragen und/oder die Detektion der Antworten auf die Fragen mit Hilfe eines Schreibbretts durchgeführt, das wie vorstehend beschrieben aus- und weitergebildet sein kann. Die Durchführung des Verfahrens kann dadurch mit einem Hilfsmittel erfolgen, das insbesondere in Pflegeinstitutionen ein übliches und normales Hilfsmittel ist, so dass eine Umstellung der beteiligten Personen nicht erforderlich ist. Hierbei wird die Erkenntnis ausgenutzt, dass in einer geriatrischen Pflegeeinrichtung beispielsweise im Gegensatz zu Hygieneanforderungen in einem Operationssaal eines Krankenhauses zur Überprüfung einer Pflege- und/oder Versorgungssituation fachärztliches Spezialwissen nicht erforderlich ist und die Übertragung von Arbeitsweisen aus dem medizinischen Bereich in den geriatrischen Bereich überraschenderweise kostengünstig und effizient erfolgen kann.

Die Erfindung betrifft ferner ein Computerprogrammprodukt zur Überprüfung und/oder Beurteilung eines durch vorgegebene Kriterien definierten Sollzustands, insbesondere einer geriatrischen und/oder palliativmedizinischen Pflege- und/oder Versorgungssituation eines Menschen, mit einer auf einem Datenträger gespeicherten maschinenlesbaren Software, die derart ausgestaltet ist, dass beim Ausführen der Software auf einem Display auf Basis der vorgegebenen Kriterien Fragen zum Istzustand ausgegeben werden, Antworten auf die Fragen detektiert und weiterverarbeitet werden, wobei bei der Weiterverarbeitung der Antworten durch einen Vergleich mit Referenzdaten die Antworten bewertet und nichtbefriedigende Antworten identifiziert werden, und im Falle einer nichtbefriedigenden Antwort mit Hilfe eines gespeicherten Expertensystems Verbesserungsvorschläge unterbreitet werden, wobei die unterbreiteten Verbesserungsvorschläge insbesondere weitere Fragen aufweisen und auf Grundlage der weiteren Antworten auf die weiteren Fragen mit Hilfe des gespeicherten Expertensystems weitere Verbesserungsvorschläge unterbreitet werden. In den Referenzdaten können die Parameter, deren Vorliegen das Erfüllen eines bestimmten Kriteriums definieren, hinterlegt sein. Hierbei kann es sich auch um Schwellwerte handeln, die zur Erfüllung eines bestimmten Kriteriums überschritten und/oder unterschritten sein müssen. Wenn die Beantwortung einer Frage dazu führt, dass ein bestimmtes Kriterium nicht erfüllt ist, kann die Antwort als nicht befriedigend bewertet werden, sofern die Antworten auf andere Fragen diese Antwort nicht aus anderen Gründen eine Bewertung als befriedigende Antwort rechtfertigen. Die von dem Expertensystem erstellten Vorschläge werden insbesondere nach Beantwortung aller Fragen bereitgestellt, um die Befragung nicht durch umgehend dargestellte Verbesserungsvorschläge zu verlangsamen. Ferner können die Antworten zu anderen Fragen Einfluss auf den zu unterbreitenden Verbesserungsvorschlag haben, so dass es möglich ist sämtliche gesammelten Informationen bei der Erstellung eines Verbesserungsvorschlags zu berücksichtigen. Die Verbesserungsvorschläge können vorzugsweise in einer abschließenden Auswertung zusammengefasst sein und beispielsweise eine ToDo-Liste von durchzuführenden Maßnahmen aufweisen.

Insbesondere ist die Software des Computerprogrammprodukts derart ausgestaltet, dass die Software eine Rechnereinheit in die Lage versetzt derartige Tätigkeiten durchzuführen. Das Computerprogrammprodukt kann insbesondere auf einem Computer und/oder einem Schreibbrett, das wie vorstehend beschrieben aus- und weitergebildet sein kann, gespeichert sein. Ferner ist es möglich, das Computerprogrammprodukt über drahtgebundene und/oder drahtlose Kommunikationsmittel auf einen dafür geeigneten Datenträger zu kopieren. Insbesondere kann das Computerprogrammprodukt als eine Signalfolge vorzugsweise elektromagnetischer Impulse vorliegen. Mit Hilfe des Computerprogrammprodukts kann die Überprüfung und/oder Beurteilung eines erreichten Sollzustands schnell, sicher und nachhaltig durchgeführt werden.

Die Erfindung betrifft ferner eine Verwendung eines Schreibbretts, das wie vorstehend beschrieben aus- und weitergebildet sein kann, zur Erfassung nicht-diagnostischer Informationen zur Pflege- und/oder Versorgungssituation von Menschen in geriatrischer und/oder palliativmedizinischer Behandlung, insbesondere zum Zustand und Ausstattung einer bewohnten Immobilie. Das Schreibbrett wird somit insbesondere nicht verwendet den körperlichen Zustand eines Menschen zu erfassen, sondern die das Wohlbefinden des Menschen betreffende Umgebung, insbesondere der Wohnraum in einer geriatrischen Pflegeeinrichtung, zu dokumentieren, um daraus Verbesserungsmöglichkeiten ableiten zu können. Durch die spezielle Ausgestaltung der mit der Schreibplatte verbundenen mindestens einen Messeinrichtung auf den speziellen Verwendungszeck im geriatrischen Bereich Informationen zu erfassen, ist es möglich die Messeinrichtung auf die zur Pflege- und/oder Versorgungssituation von Menschen in geriatrischer und/oder palliativmedizinischer Behandlung relevanten Daten zu beschränken, die sich beispielsweise aus einem vorgegebenen Kriterienkatalog ergeben. Insbesondere kann die Messeinrichtung dahingehend ausgestaltet sein, dass lediglich die Erfüllung beziehungsweise Nicht-Erfüllung eines bestimmten Kriteriums erfasst wird. Insbesondere wird durch die Messeinrichtung nicht eine zu messende Größe quantitativ ermittelt sondern lediglich qualitativ mit einem Soll-Wert verglichen. Dies vereinfacht den Aufbau der Messeinrichtung und erleichtert die Auswertung der erfassten Informationen, wodurch eine schnelle und sichere Überprüfung und/oder Beurteilung eines durch vorgegebene Kriterien definierten Sollzustands einer Pflege- und/oder Versorgungssituation von Menschen in geriatrischer und/oder palliativmedizinischer Behandlung ermöglicht ist.

Nachfolgend wird die Erfindung unter Bezugnahme auf die anliegenden Zeichnungen anhand eines bevorzugten Ausführungsbeispiels exemplarisch erläutert. Es zeigen:
- Fig. 1:: eine schematische Rückansicht eines Schreibbretts und
- Fig. 2:: eine schematische Seitenansicht des Schreibbretts aus Fig. 1.

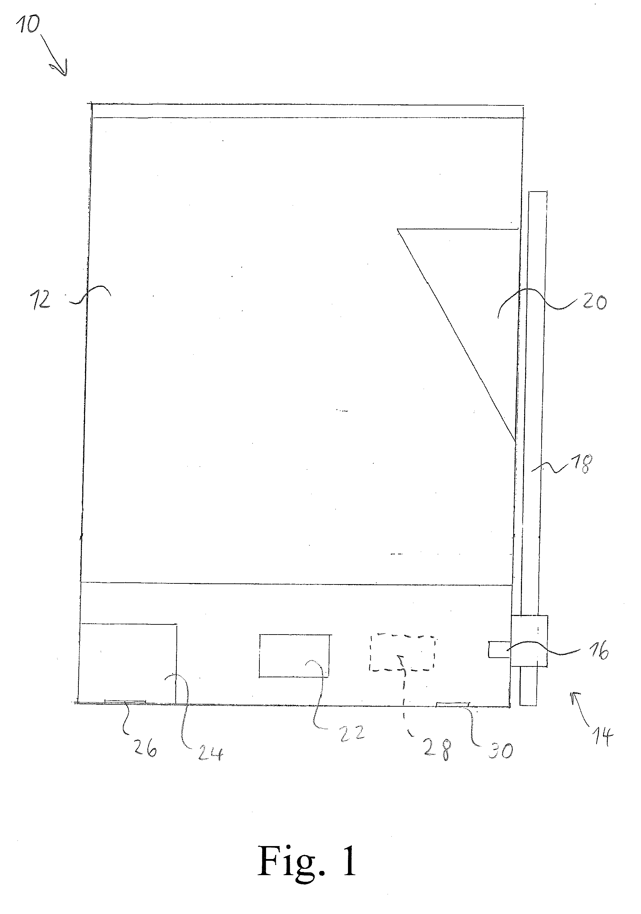

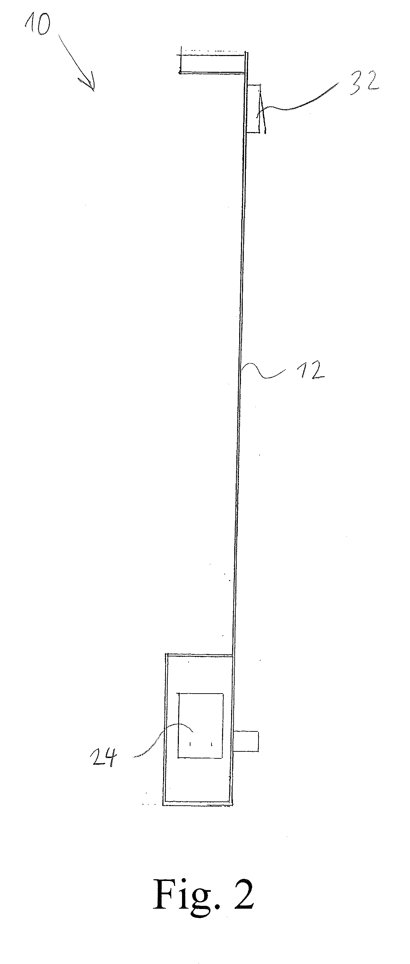

Das in Fig. 1 dargestellte Schreibbrett 10 weist eine Schreibplatte 12 auf, die eine Schreibfläche bereitstellen kann, wobei die Schreibfläche auch durch ein Display, insbesondere Touchscreen, ausgebildet werden kann. Das Schreibbrett 10 weist eine Längenmesseinrichtung 14 auf, die einen über ein Gelenk 16 schwenkbar mit der Schreibplatte 12 verbundenen Stab 18 aufweist. Durch den Stab 18 wird ein definierter Mindestabstand zwischen einer Oberkante eines Bettgitters eines Bewohnerbetts zu einer Oberseite einer Matratze eines Bewohnerbetts vorgegeben. Damit die Schreibplatte 12 möglichst rechtwinklig an der Oberkante des Bettgitters angesetzt werden kann, kann ein Ansatz 20 von der Schreibplatte 12 abgeklappt werden, der in der ausgeklappten Gebrauchsposition zwischen dem Ansatz 20 und der Schreibplatte 12 einen rechten Winkel ausbildet. Das Klemmbrett 10 kann ferner ein digitales Thermometer 22 aufweisen, das die aktuelle Temperatur anzeigen kann. Insbesondere kann zusätzlich ein Feuchtigkeitsmesser vorgesehen sein, der die Luftfeuchtigkeit anzeigen kann. Ferner können weitere Messeinrichtungen vorgesehen sein, wie beispielsweise ein Fotometer zur Helligkeitsmessung, und/oder ein Lautstärkemesser zur Lautstärkemessung. Das dargestellte Schreibbrett 10 weist ferner einen Scanner 24 auf, der über ein Scannerfenster 26 Barcodes einlesen kann. Die Messergebnisse der entsprechenden Messeinrichtungen können mit Hilfe einer Rechnereinheit 28 verarbeitet und insbesondere über einen Transmitter 30, vorzugsweise automatisch, an einen Server übertragen werden. Zusätzlich oder alternativ können die Messergebnisse über ein nicht dargestelltes Display von der Schreibplatte 12 dargestellt werden. Mit Hilfe dieses Displays können auch Fragen zur Pflege- und/oder Versorgungssituation eines Menschen ausgegeben werden, wobei mit Hilfe der Eingabemittel die Antworten auf die Fragen detektiert und vorzugsweise in der Rechnereinheit 28 verarbeitet werden. Zusätzlich oder alternativ können die abzuarbeitenden Fragen mit Hilfe einer Klemme 32 (Fig. 2) mit dem Schreibbrett 12 verklemmt und schriftlich beantwortet werden. Ferner ist es möglich mit der Klemme 32 Notizzettel zu befestigen, um in Reaktion auf die Ergebnisse der Überprüfung der Pflege- und/oder Versorgungssituation durchzuführende Maßnahmen oder sonstige Hinweise zu notieren.

## Patentansprüche

1. Schreibbrett, insbesondere Klemmbrett, zur manuellen Informationsspeicherung mit einer Schreibplatte (12) zur Bereitstellung einer Schreibfläche und mindestens einer mit der Schreibplatte (12) verbundenen Messeinrichtung (14, 22, 24) zur Ermittlung von Pflegedaten und/oder Versorgungsdaten für die Pflege- und/oder Versorgungssituation von Menschen, insbesondere Menschen in geriatrischer und/oder palliativmedizinischer Behandlung.

2. Schreibbrett nach Anspruch 1 **dadurch gekennzeichnet, dass** eine Messeinrichtung als Längenmesseinrichtung (14) zur Detektion eines Abstands zwischen einer Oberkante eines Bettgitters eines Bewohnerbetts zu einer Oberseite einer Matratze des Bewohnerbetts ausgestaltet ist, wobei die Längenmesseinrichtung (24) insbesondere als schwenkbar mit der Schreibplatte (12) verbundene Lehre ausgestaltet ist.

3. Schreibbrett nach Anspruch 1 oder 2 **dadurch gekennzeichnet, dass** mit der Schreibplatte (12) ein zwischen einer eingeklappten Ruheposition und einer im Wesentlichen rechtwinkelig abstehenden Gebrauchsposition bewegbarer Ansatz (20) verbunden ist, wobei der Ansatz (20) insbesondere in der Ruheposition reibschlüssig und/oder formschlüssig feststellbar ist.

4. Schreibbrett nach einem der Ansprüche 1 bis 3 **dadurch gekennzeichnet, dass** eine Messeinrichtung als Thermometer (22) zur Messung der Temperatur eines Bewohnerzimmers und/oder als Feuchtigkeitsmesser zur Luftfeuchtigkeitsmessung des Bewohnerzimmers ausgestaltet ist.

5. Schreibbrett nach einem der Ansprüche 1 bis 4 **dadurch gekennzeichnet, dass** eine Messeinrichtung als Photometer zur Helligkeitsmessung eines Bewohnerzimmers ausgestaltet ist.

6. Schreibbrett nach einem der Ansprüche 1 bis 5 **dadurch gekennzeichnet, dass** eine Messeinrichtung als Lautstärkemesser zur Lautstärkemessung eines Bewohnerzimmers ausgestaltet ist.

7. Schreibbrett nach einem der Ansprüche 1 bis 6 **dadurch gekennzeichnet, dass** mit der Schreibplatte (12) eine Leseeinheit, insbesondere Barcode-Scanner (24), zur Identifikation von im Bewohnerzimmer vorgesehenen Hilfsmitteln verbunden ist, wobei die Hilfsmittel jeweils mit einem von der Leseeinheit lesbaren Code versehen sind.

8. Schreibbrett nach einem der Ansprüche 1 bis 7 **dadurch gekennzeichnet, dass** die Schreibplatte (12) ein Display, insbesondere Touchscreen, aufweist, wobei das Display mit einer Rechnereinheit (28) verbunden ist.

9. Schreibbrett nach Anspruch 8 **dadurch gekennzeichnet, dass** die Rechnereinheit (28) mit der mindestens einen Messeinrichtung (14, 22, 24) zur Darstellung der Messergebnisse auf dem Display und/oder zur Verarbeitung der Messergebnisse verbunden ist.

10. Schreibbrett nach Anspruch 8 oder 9 **dadurch gekennzeichnet, dass** die Rechnereinheit (28) ein Computerprogrammprodukt zur Pflegeüberprüfung und/oder Pflegebeurteilung aufweist, wobei das Computerprogrammprodukt derart ausgestaltet ist, dass beim Ausführen des Computerprogrammprodukts auf dem Display Fragen zur Pflege- und/oder Versorgungssituation eines Menschen ausgegeben werden,
Antworten auf die Fragen detektiert und weiterverarbeitet werden, wobei bei der Weiterverarbeitung der Antworten durch einen Vergleich mit Referenzdaten die Antworten bewertet und nicht befriedigende Antworten identifiziert werden, und
im Falle einer nicht befriedigenden Antwort mit Hilfe eines gespeicherten Expertensystems Verbesserungsvorschläge unterbreitet werden, wobei die unterbreiteten Verbesserungsvorschläge insbesondere weitere Fragen aufweisen und auf Grundlage der weiteren Antworten auf die weiteren Fragen mit Hilfe des gespeicherten Expertensystems weitere Verbesserungsvorschläge unterbreitet werden.

11. Schreibbrett nach einem der Ansprüche 8 bis 10 **dadurch gekennzeichnet, dass** die Rechnereinheit (28) mit einem Transmitter (30) zur drahtlosen Kommunikation erhaltener Daten mit einer Servereinheit verbunden ist, wobei insbesondere die Rechnereinheit zur permanentspeicherlosen Übertragung der erhaltenen Daten mit dem Transmitter (30) verbunden ist.

12. Verfahren zur Überprüfung und/oder Beurteilung eines durch vorgegebene Kriterien definierten Sollzustands, insbesondere einer geriatrischen und/oder palliativmedizinischen Pflege- und/oder Versorgungssituation eines Menschen, bei dem auf einem Display auf Basis der vorgegebenen Kriterien Fragen zum Istzustand ausgegeben werden,
Antworten auf die Fragen detektiert und weiterverarbeitet werden, wobei bei der Weiterverarbeitung der Antworten durch einen Vergleich mit Referenzdaten die Antworten bewertet und nicht befriedigende Antworten identifiziert werden, und
im Falle einer nicht befriedigenden Antwort mit Hilfe eines gespeicherten Expertensystems Verbesserungsvorschläge unterbreitet werden, wobei die unterbreiteten Verbesserungsvorschläge insbesondere weitere Fragen aufweisen und auf Grundlage der weiteren Antworten auf die weiteren Fragen mit Hilfe des gespeicherten Expertensystems weitere Verbesserungsvorschläge unterbreitet werden.

13. Verfahren nach Anspruch 12, bei dem zur Überprüfung und/oder Beurteilung des Sollzustands relevante Messdaten mit Hilfe eines Schreibbretts (10) nach einem der Ansprüche 1 bis 11 ermittelt werden und/oder die Ausgabe der Fragen und/oder die Detektion der Antworten auf die Fragen mit Hilfe eines Schreibbretts (10) nach einem der Ansprüche 1 bis 11 durchgeführt werden.

14. Computerprogrammprodukt zur Überprüfung und/oder Beurteilung eines durch vorgegebene Kriterien definierten Sollzustands, insbesondere einer geriatrischen und/oder palliativmedizinischen Pflege- und/oder Versorgungssituation eines Menschen, mit einer auf einem Datenträger gespeicherten maschinenlesbaren Software, die derart ausgestaltet ist, dass bei einem Ausführen der Software
auf einem Display auf Basis der vorgegebenen Kriterien Fragen zum Istzustand ausgegeben werden,
Antworten auf die Fragen detektiert und weiterverarbeitet werden, wobei bei der Weiterverarbeitung der Antworten durch einen Vergleich mit Referenzdaten die Antworten bewertet und nicht befriedigende Antworten identifiziert werden, und
im Falle einer nicht befriedigenden Antwort mit Hilfe eines gespeicherten Expertensystems Verbesserungsvorschläge unterbreitet werden, wobei die unterbreiteten Verbesserungsvorschläge insbesondere weitere Fragen aufweisen und auf Grundlage der weiteren Antworten auf die weiteren Fragen mit Hilfe des gespeicherten Expertensystems weitere Verbesserungsvorschläge unterbreitet werden.

15. Verwendung eines Schreibbretts (10) nach einem der Ansprüche 1 bis 11 zur Erfassung nicht-diagnostischer Informationen zur Pflege- und/oder Versorgungssituation von Menschen in geriatrischer und/oder palliativmedizinischer Behandlung, insbesondere zum Zustand und Ausstattung einer bewohnten Immobilie.
